# EUROPEAN PATENT SPECIFICATION

(11) **EP 1 132 059 B1**
(45) Date of publication and mention of the grant of the patent: **02.03.2005**
(21) Application number: 01302054.0
(22) Date of filing: 06.03.2001
(51) Int. Cl.: A61F 2/06

(54) **Balloon catheter with balloon shoulders**
Ballonkatheter mit Schultern auf dem Ballon
Cathéter à ballonnet avec épaulements sur le ballonnet

(30) Priority: 07.03.2000 US 520258
(43) Date of publication of application: 12.09.2001
(73) Proprietor: Cordis Corporation, Miami Lakes Florida 33014 (US)
(72) Inventor: Johnson, Eric G., Flagstaff, AZ 86001 (US); Weigand, Condrad T., Coral Springs, Florida 33065 (US)
(74) Representative: Mercer, Christopher Paul

(56) References cited:
- WO-A-97/21400
- US-A- 5 935 135
- US-A- 6 027 510

## Description

The present invention relates generally to medical devices, and more particularly to a balloon catheter and stent delivery system.

Balloon catheters are used in a variety of therapeutic applications, including intravascular catheters for procedures such as angioplasty treating coronary, neurological and peripheral blood vessels partially or totally blocked or narrowed by a stenosis. By way of example, the present invention will be described in relation to coronary and peripheral angioplasty treatments. However, it should be understood that the present invention relates to balloon catheters and stent delivery systems generally, and is not limited to angioplasty.

Most balloon catheters have a relatively long and flexible tubular shaft defining one or more passages or lumens, and an inflatable balloon attached near one end of the shaft. This end of the catheter where the balloon is located is customarily referred to as the "distal" end, while the other end is called the "proximal" end. The balloon is connected to one of the lumens extending through the shaft to selectively inflate and deflate the balloon. The other end of this inflation lumen leads to a hub coupling at the other end for connecting the shaft lumens to various equipment. Examples of this type of balloon catheter are shown in U.S. Patent number 5,304,197, entitled "Balloons For Medical Devices And Fabrication Thereof," issued to Pinchuk et al. on April 19, 1994, and also in U.S. Patent number 5,370,615, entitled "Balloon Catheter For Angioplasty," issued to Johnson on December 6, 1994.

A common treatment method for using such a balloon catheter is to advance the catheter into the body of a patient, by directing the catheter distal end percutaneously through an incision and along a body passage until the balloon is located within the desired site. The term "desired site" refers to the location in the patient's body currently selected for treatment by a health care professional. A larger guiding catheter may often be used to access the local area near the desired site, providing a smooth, supported lumen for conducting other devices including balloon catheters to the desired site. After the balloon is within the desired site, it can be selectively inflated to press outward on the body passage at relatively high pressure to a relatively constant diameter, in the case of an inelastic or non-compliant balloon material.

This outward pressing of a constriction or narrowing at the desired site in a body passage is intended to re-open or dilate that body passageway or lumen, increasing its inner diameter or cross-sectional area. In the case of a blood vessel, this procedure is referred to as angioplasty. The narrowing of the body passageway lumen is called a lesion or stenosis, and may be formed of hard plaque or viscous thrombus. The objective of this procedure is to treat the lesion by increasing the cross-sectional area of the blood vessel, to encourage greater blood flow through the newly expanded vessel.

Unfortunately, the lumen at the angioplasty site may re-close or become narrow again. This phenomenon is called restenosis, and may occur in a certain percentage of percutaneous transluminal angioplasty patients. Restenosis may require an additional procedure, such as another angioplasty, drug therapy treatment, or even surgery including bypass graft.

In an effort to prevent restenosis, a short flexible cylinder or scaffold made of metal or polymers, referred to as a stent, may be permanently implanted into the vessel to hold the lumen open, to reinforce the vessel wall and improve blood flow. In 1998, coronary stents were placed in an estimated half million patients in the United States. The presence of a stent tends to keep the blood vessel open longer, but their use may be limited by various factors, including size and location of the blood vessel, a complicated or tortuous vessel pathway, etc. Also, even a vessel with a stent may eventually develop restenosis.

One type of stent is expanded to the proper size by inflating a balloon catheter, referred to as "balloon-expandable" stents. Balloon-expandable stents are crimped or compressed to a diameter during delivery that is smaller than the eventual deployed diameter at the desired site. When positioned at the desired site within the lesion, they are deployed by inflating a balloon into the desired diameter.

Friction forces may tend to cause a crimped stent to slip in a proximal direction while the catheter system is advanced, or to slip in a distal direction if the physician decides to withdraw the stent without deploying it. In some situations, a stent may tend to possibly catch on a distal end of a guiding catheter. It is of course desirable to retain the stent in the proper position, during advancement along a vascular path to the desired site, and subsequent removal if necessary.

In addition, it is desirable to provide a stent delivery system more capable of advancing a crimped stent across a previously deployed stent, or possibly withdrawing it into a guiding catheter. It is also desirable to provide a balloon having a design capable of acting as a dilator or having a self-centering capability.

In US 5,935,135, there is disclosed a balloon catheter stent deployment system of the type set forth in the preamble to the accompanying claim 1. In WO 97/21400, there is disclosed another balloon catheter stent deployment system of the type set forth in the preamble to the accompanying claim 1.

Accordingly, it is an object of the present invention to provide balloon catheter systems for enhanced stent position retention during longitudinal movement of the catheter

According to the invention, there is provided a balloon catheter stent deployment system of the type set forth in the accompanying claim 1.

These and various other objects, advantages and features of the invention will become apparent from the following description and claims, when considered in conjunction with the appended drawings.
Figure 1 is an external perspective view of a balloon catheter having a stent mounted around the balloon, arranged according to the principles of the present invention;
Figure 2 is a longitudinal cross-section view of a balloon catheter according to the principles of the present invention;
Figure 3 is a longitudinal cross-section view of a balloon catheter and assembly equipment;
Figure 4 is a partial longitudingal cross-section view of the balloon catheter distal end and the stent of Figure 1;
Figure 5 is a partial longitudinal cross-section view of a stent and a balloon having the shoulders of the present invention;
Figure 6 is a partial longitudinal cross-section view of a balloon catheter distal end and a stent, showing an inflated balloon; and
Figure 7 is a longitudinal cross-section view of a balloon catheter, stent, and assembly equipment.

The following description of the preferred embodiments of the present invention is merely illustrative in nature, and as such it does not limit in any way the present invention, its application, or uses. Numerous modifications may be made by those skilled in the art without departing from the scope of the invention.

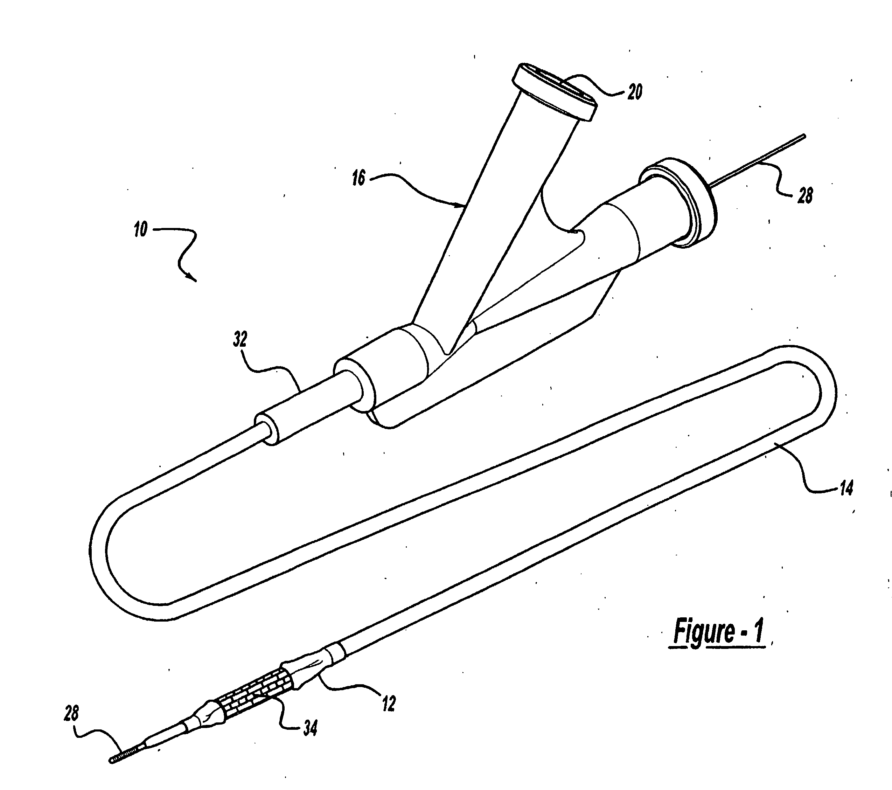

Referring to the drawings, a balloon catheter system is depicted, with one of the preferred embodiments of the present invention being shown generally at 10. The balloon catheter of Figure 1 has an inflatable balloon 12, a relatively long and flexible tubular shaft 14, and a hub 16. The balloon 12 is affixed to the shaft 14 near a distal end of the shaft 14, and the hub 16 is affixed to the proximal end of the shaft 14.

The shaft 14 defines one or more passages or lumens extending through the shaft, at least one of which is an inflation lumen 18 connected to the balloon 12 for the purpose of selectively inflating and deflating the balloon 12. The inflation lumen 18 thus provides fluid communication between the interior of the balloon 12 at the distal end of the inflation lumen 18, and a hub inflation port 20 having a coupling or luer-lock fitting at the proximal end for connecting the inflation lumen 18 to a source of pressurized inflation fluid (not shown) in the conventional manner.

In the illustrated embodiment, the shaft 14 is constructed of an inner and outer tubular body 22 and 24. The inner body 22 defines a guidewire lumen 26, while the inflation lumen 18 is defined by the annular space between the inner and outer tubular bodies 22 and 24. The guidewire lumen 26 is adapted to receive an elongated flexible guidewire 28 in a sliding fashion, such that the guidewire 28 and catheter 10 may be advanced or withdrawn independently, or the catheter 10 may be guided along a path selected with the guidewire 28. The shaft 14 may of course have various configurations instead of this coaxial design, including a single extruded tube defining any suitable number of parallel side-by-side lumens, a proximal shaft portion formed of a metal hypotube, and other designs.

The proximal hub 16 is affixed to the proximal end of the shaft 14, and provides an inflation port 20 and a guidewire port 30, again with a luer-lock fitting or hemostatic valve. Such a valve allows the guidewire 28 to traverse and slide within the guidewire lumen 26, yet while resisting the loss of blood or other fluids through the guidewire lumen 26 and guidewire port 30. As shown in the drawings, the inner and outer tubular bodies 22 and 24 are securely received within the hub 16, and surrounded by a tubular strain relief 32. The hub 16 provides fluid communication between the guidewire lumen 26 and a guidewire port 30 and coupling, as well as between the annular inflation lumen 18 and the inflation port 20 and coupling.

A stent 34 of any suitable type or configuration may be provided with a catheter 10 of the present invention, such as the well-known Palmaz-Schatz balloon expandable stent. Various kinds and types of stents are available in the market, and many different currently available stents are acceptable for use in the present invention, as well as new stents which may be developed in the future. The stent 34 depicted in the drawings is a cylindrical metal mesh stent having an initial crimped outer diameter, which may be forcibly expanded by the balloon to a deployed diameter. When deployed in a body passageway of a patient; the stent may be designed to preferably press radially outward to hold the passageway open.

As shown in the drawings, the balloon 12 in its fully inflated profile shape has a cylindrical working portion 36 with an inflated diameter located between a pair of conical end portions 38, and a pair of proximal and distal legs 48 and 50 affixed to the shaft 14. The balloon 12 in its deflated profile shape preferably has several pleats that are wrapped around the shaft. The balloon pleats are illustrated in Figure 1, but are omitted from the other drawings for the sake of clarity. The balloon material is preferably substantially inelastic, and stretches a relatively small amount under pressures of 15.195 bar (15 atmospheres) or more. Various different materials may be used, including Nylon, PEEK, a nylon material sold under the trade name Pebax, or a block copolymer thereof.

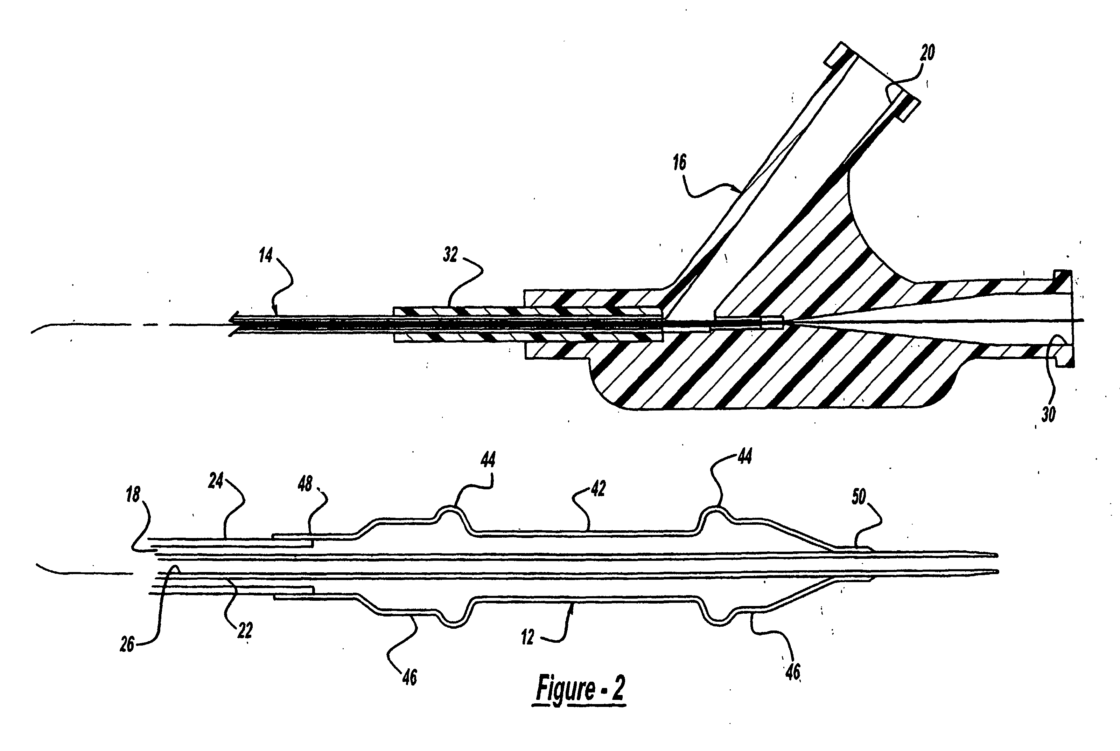

The novel balloon catheter system of the present invention provides several advantages. Among these advantages is that the balloon has a pair of round annular shoulders immediately adjacent the proximal and distal ends of the stent, as well as a pair of cylindrical transition portions extending proximally and distally of the shoulders. The shoulders have a diameter greater than the crimped stent, thus protecting the stent and minimizing any possibility of the stent moving due to friction. The shoulders and transition portions tend to act as dilators, to encourage easy advancement through challenging anatomy or a previously deployed stent, or withdrawal into the guiding catheter. The balloon also has a composite profile shape which varies at different pressures. The balloon initially is in a deflated state and has a deflated profile shape, as specifically illustrated in Figure 2, having a central bed portion 42 with a deflated bed diameter being flanked by a pair of proximal and distal shoulders 44 defining deflated shoulder diameters that are larger than the deflated bed diameter.

The balloon shoulders 44 are flanked by a pair of cylindrical transition portions 46 extending in proximal and distal directions respectively, and which taper smoothly down to a proximal and distal cylindrical balloon leg 48 and 50. The proximal balloon leg 48 is affixed to the outer tube 24, while the distal balloon leg 50 is affixed to the inner tube 22. This deflated balloon profile shape thus provides a bed or nest portion 42 for receiving the stent 34 and tending to hold the stent 34 in place, while minimizing friction or adverse contact between the ends of the stent 34 and a blood vessel wall. The present invention thus tends to protect the leading or distal ends of the stent 34 during advancement into the patient's body, and the proximal end of the stent 34 during any withdrawal of the catheter system.

It is also possible that the shoulders of the present invention will tend to resist any tendency of the stent to shift position during actual inflation, in addition to during longitudinal movement of the catheter.

Another possible advantage is that the stent delivery system may tend to center itself during advancement through any previously deployed stent, or if the stent delivery system is pulled back into the guiding catheter. This tendency may reduce any possibility of a proximal or distal edge of the stent from catching on a distal end of a guiding catheter or a proximal end of a prior stent, respectively.

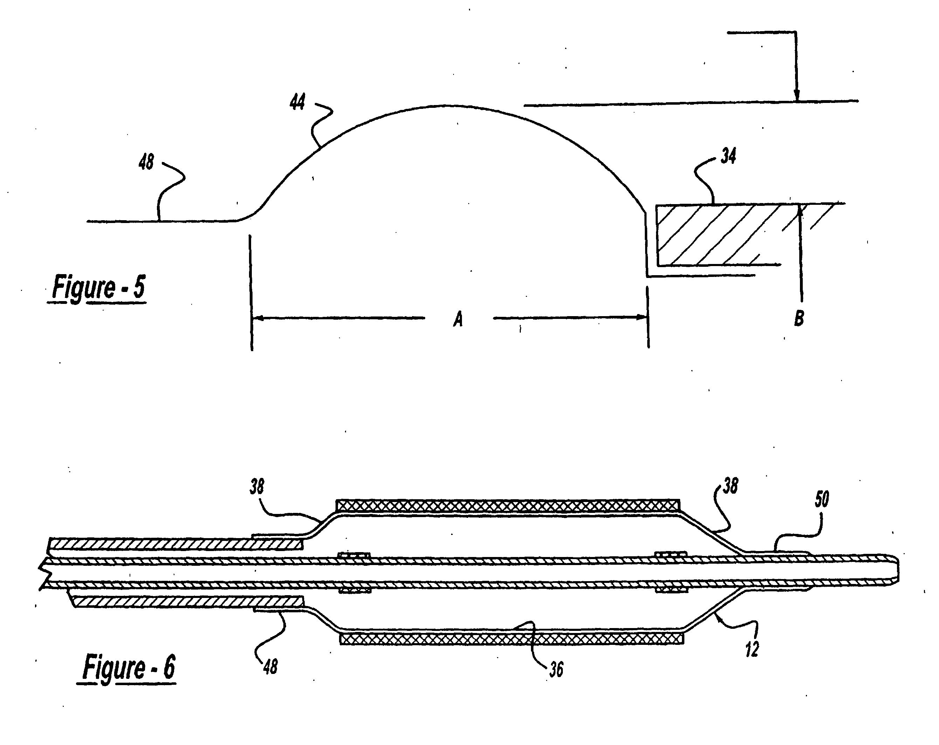

Figure 6 depicts the balloon 12 in its fully inflated profile shape. The stent bed shape disappears, and the balloon profile shape changes or morphs into a different profile shape when inflated at full inflation pressure. This fully inflated shape provides the preferable cylindrical working portion 36, wherein the portion of the balloon supporting and expanding the stent 34 has an inflated diameter larger than any other portion of the balloon 12. This feature tends to prevent any part of the balloon from expanding excessively, which might cause local trauma to the blood vessel wall.

Accordingly, some portions of the present balloon reverse positions during inflation. The central bed portion 42 initially has a smaller deflated diameter than the proximal and distal shoulders 44, which provides a desirably small outer maximum diameter for ease of insertion. The initial outer maximum diameter is referred to as the "primary profile." In contrast, the central balloon portion 42 expands on full inflation to the largest diameter of the balloon 12, while the portions that previously formed the balloon shoulders 44 expand comparably less. Indeed, the former shoulders 44 and transition portion 46 define the proximal and distal end conical portions 38 of the fully inflated profile shape.

In the deflated shape, the balloon is therefore temporarily reformed into a different shape than what might conventionally result from simply deflating and pleating a previously known balloon. This temporarily reformed shape enhances stent position retention, and yet exhibits the preferable fully inflated shape.

Another advantage of the present invention is the absence of any type of physical collar or other retaining device within the balloon, or on the outer balloon surface, or mounted on the balloon catheter shaft, which might undesirably increase the primary and/or secondary profiles of the stent delivery system.

An additional possible advantage of the present invention is that a physician may be able to use the balloon shoulders to "feel" for and confirm the dimensions of a lesion or stenosis. This tactile confirmation may enable the physician to confirm a correct centered placement of the stent in a lesion, or possibly even determine if a selected stent length is the wrong size for a particular lesion.

The dimensions that may be preferred for the present invention will of course vary, as the device is sized to a patient's vascular anatomy. The length of the shoulders 44 (dimension "A" in Figure 5) may be approximately 0.381-1.27 mm (0.015-0.050 inches) for a coronary balloon catheter. The shoulders 44 are also preferably larger than the crimped outer diameter of the stent 34, such that dimension "B" in Figure 5 may be approximately 0.051-0.254mm (0.002-0.010 inches).

The balloon catheter system of the present invention may be made using any of the following methods, as well as various modifications that will be apparent to those skilled in the art. The balloon is folded into any suitable or preferable number of longitudinal pleats which are wrapped around a portion of the catheter shaft, either manually or by using a pleating machine.

The balloon is then temporarily held in its pleated condition by slipping a forming tube in the proximal direction onto the pleated balloon, while the assembly is transported to the next processing station. After the forming tube is removed, a stent is slipped onto the pleated balloon. The stent is then gently crimped or compressed around the balloon, with the pleats intact, to a crimped condition in which the stent has a crimped outer diameter.

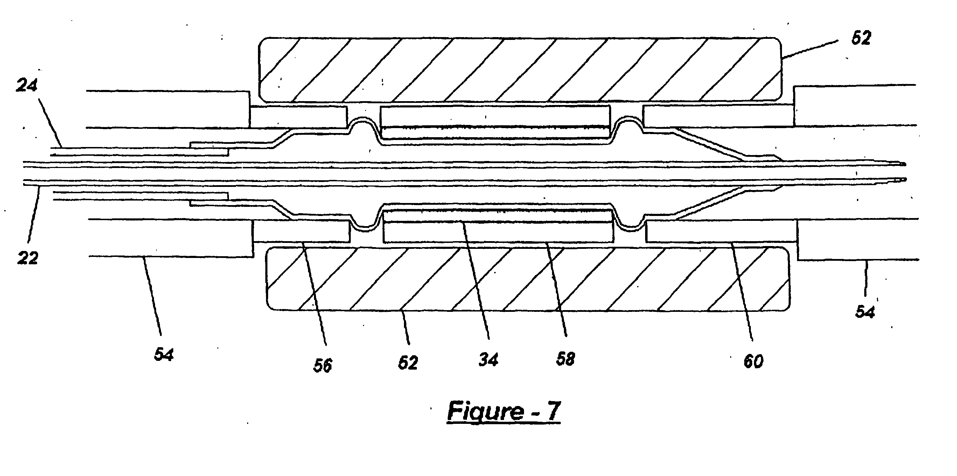

As shown in Figure 7, the resulting balloon catheter and stent assembly is then placed in a tubular mold having a pair of splits, and having an internal diameter the same as or slightly larger than the crimped outer diameter of the stent. The tubular mold should have a constant inner diameter, to cause the balloon shoulders to have the preferred shape and diameter.

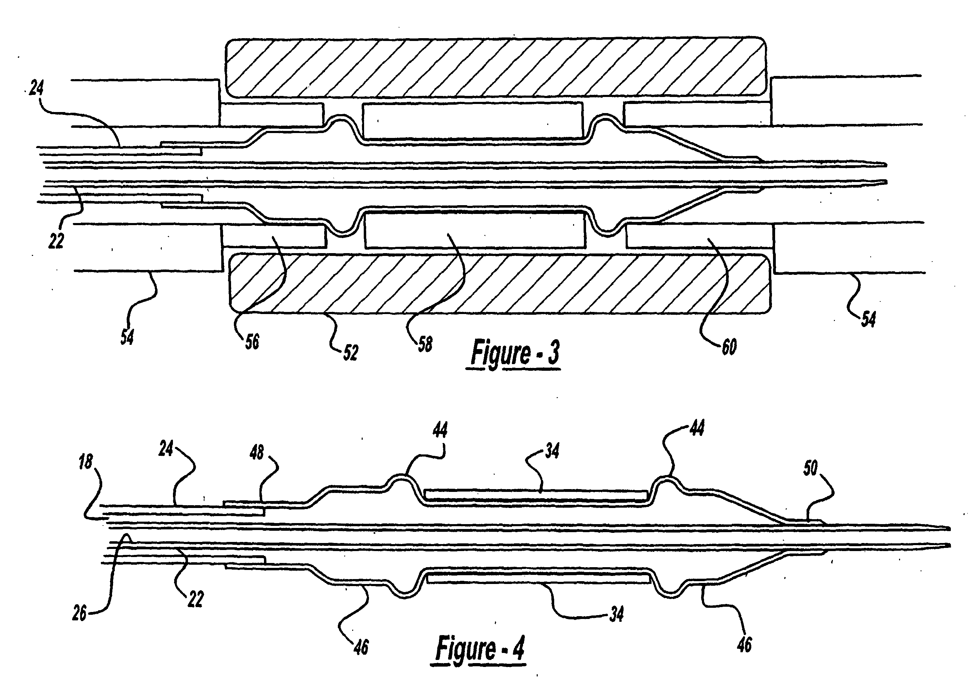

An alternative configuration is depicted in Figure 3, showing a system for making a balloon catheter alone, without a stent, in accordance with the present invention. The tubular mold may be provided with the constant-diameter proximal, central, and distal tubes 56, 58 and 60 shown in Figure 7, or with varying diameter tubes as shown in Figure 3.

The balloon is then pressurized by applying a pressurized fluid to the inflation port and through the inflation lumen. The preferred pressure of the inflation within the tubular mold may slightly exceed the rated burst pressure of the balloon, and the mold will prevent expansion of the stent while allowing the proximal and distal balloon shoulders to form. The pressurized fluid may preferably be dry nitrogen, and the pressure may preferably be maintained for a preselected period of time.

The mold may be formed as shown in Figure 3, with a proximal, central, and distal PTFE tube 56, 58 and 60 respectively. While the mold with the accompanying balloon catheter and stent assembly is held under pressure, they are then held in a hot box or heated die 52. The heat tends to set the stent in place, thus forming the desired proximal and distal shoulders 44. Of course, a hot air or liquid system may also be used. The preferred temperature of the heating system is preferably below the permanent deformation temperature of the balloon material, and the time and pressure of this process may be extended to ensure that such a temperature will result in the desired composite shape and temporary reformation of the balloon.

The pressure is released and the balloon and stent assembly are then removed from the mold and heating system. In the alternative, the pressure may be maintained as the balloon and stent assembly is allowed to cool for a period of time.

Several features of this preferred method of making the balloon catheter stent delivery system of the present invention have effect on the performance of the resulting product, including the temperatures, pressures, time periods, crimped outer diameter of the stent, the internal diameter of the mold, as well as the thermal characteristics of the balloon, stent and mold.

The particular method described above for making a balloon catheter stent delivery system obviously produces a balloon catheter having an already mounted stent. However, the methods of the present invention may also be used to produce a balloon catheter having the desired features without incorporating an included stent. Accordingly, the physician may then install and manually crimp a selected stent having the proper dimensions, while yet taking advantage of the enhanced stent position retention and safety features of the present invention.

Accordingly, a balloon catheter having enhanced stent position retention may be made, without requiring a stent during the process, by a method similar to that described above. During this modified method, the presence of a stent is obviated by replacing it with a 'phantom stent.' One advantage of using a phantom stent is that it costs much less than an actual metal stent. Such a phantom stent may be formed of any suitable plastic material capable of withstanding the temperatures and pressures of the manufacturing method without melting or deforming. Suitable plastic materials for the phantom stent may thus include for example, PTFE or polyethylene.

Since the plastic phantom stent will not crimp in the same way that an actual metal stent does, it may be provided with a longitudinal slit or preferably a spiral cut. The phantom stent may thus be installed onto the pleated balloon, and removed after forming the stent nest with the accompanying shoulders, by way of the cut in the plastic material. In the alternative, the shoulders may be formed large and resilient enough that they will survive if a tubular phantom stent without an access cut is simply pulled off.

When a balloon catheter is made and processed according to the methods of the present invention, and then the phantom stent is removed, the resulting balloon catheter has protruding round shoulders and a stent nest or bed portion which will provide enhanced stent position retention for any stent of suitable dimensions.

It should be understood that an unlimited number of configurations for the present invention could be realized. The foregoing discussion describes merely exemplary embodiments illustrating the principles of the present invention, the scope of which is recited in the following claims. Those skilled in the art will readily recognize from the description, claims, and drawings that numerous changes and modifications can be made without departing from the scope of the invention as defined in the accompanying claims.

## Claims

1. A balloon catheter stent deployment system, comprising:
a balloon catheter (10) with an elongated flexible shaft (14) having proximal and distal ends, a hub (16) affixed to the shaft proximal end and having an inflation port (20), and an inflatable balloon (12) affixed to the shaft near the shaft distal end; the shaft defining an inflation lumen (18) providing fluid communication of an inflation fluid between the hub inflation port (20) and the balloon (12); and
an expandable tubular mesh stent (34) defining a tubular wall thickness, mounted around the balloon and being crimped in an initial state to an initial crimped outer diameter, the stent having substantially no tendency to self-expand absent an expansive force caused by inflating the balloon (12);
wherein the balloon (12) is adapted for selective inflation from a deflated state to an inflated state, as well as later deflation; the balloon being formed of a substantially inelastic material; the balloon in the inflated state having a cylindrical working portion (36) with an inflated cross-sectional area located between a pair of cylindrical leg portions (48, 50), the legs being affixed to the shaft (14); the balloon being initially in the deflated state having multiple longitudinal pleats; thereby tending to frictionally retain the stent (34) in an initial longitudinal position while the catheter system is advanced or withdrawn along a vascular path;
wherein the balloon (12) in its deflated state defines a central cylindrical bed portion (42) underneath the crimped stent (34), and defines a first and a second balloon shoulder (44) located immediately proximal and distal of the stent and balloon bed portion (42) ; each balloon shoulder (44) in an initial deflated state having an annular shape with an arcuate wall annular cross-section and an initial outer diameter greater than the initial outer diameter of the stent, thereby tending to frictionally retain the stent (34) in an initial longitudinal position while the catheter system is advanced or withdrawn along a vascular path and while the balloon (12) is inflated; such that the balloon defines an indented bed shape (42) for the stent in the deflated state; wherein said shoulders (44) in the deflated state are adapted to protect the proximal and distal ends of the stent and reduce the possibility of undesirable contact between the proximal and distal ends of the stent and a sidewall of the vascular path; and
wherein the balloon (12) is adapted to inflate and expand the stent (34) to a larger deployed stent diameter, whereby the balloon forms said cylindrical working portion (36) and said inflated state above a preselected transition pressure;
**characterised in that** the balloon (12) in the deflated state defines a pair of cylindrical transition portions (46) immediately proximal and distal of the shoulders (44), and defines a pair of tapers (38) extending from the transition portions (46) to the balloon legs (48, 50); and the balloon (12) in the inflated state includes a pair of conical portions (38) between which the cylindrical working portion (36) is located.

2. The balloon catheter stent deployment system of Claim 1, wherein said stent (34) is formed of an integral unitary metal cylinder having a plurality of apertures for allowing the stent to expand from the initial diameter to the deployed diameter.

3. The balloon catheter stent deployment system of Claim 1, wherein the stent (34) is formed of one or more wires arranged into an integral non-unitary metal cylinder having one or more attachments between selected portions of the wires.

4. The balloon catheter stent deployment system of Claim 1, 2 or 3, further comprising a guidewire lumen (26) adapted to slidably receive an elongated flexible guidewire (28); the guidewire lumen extending continuously from a distal guidewire port defined by the catheter distal of the balloon to a proximal guidewire port (30) near the proximal end of the shaft (14), in an over-the-wire configuration.

5. The balloon catheter stent deployment system of Claim 1, 2, or 3, further comprising a guidewire lumen (26) adapted to slidably receive an elongated flexible guidewire (28); the guidewire lumen extending continuously from a distal guidewire port defined by the catheter distal of the balloon to a proximal guidewire port (30) at a point nearer to the catheter distal end than the catheter proximal end, in a rapid exchange configuration.

6. The balloon catheter stent delivery system of any preceding claim, wherein the balloon shoulders (44) and transition portions (46) tend to act as dilators and encourage smooth advancement along a vascular path.

7. The balloon catheter stent deployment system of any preceding claim, wherein the balloon shoulders (44) tend to center the balloon (12) and stent (34) when crossing a previously implanted stent and facilitate such crossing.

8. The balloon catheter stent deployment system of any preceding claim, wherein the balloon shoulders (44) tend to center the balloon (12) and stent (34) if withdrawing the stent back into a guiding catheter.

9. The balloon catheter stent deployment system of any preceding claim, wherein the shoulders (44) are operative to provide a tactile confirmation of the position and size of a stenosis.

## Patentansprüche

1. Ballonkatheterstententfaltungssystem umfassend:
einen Ballonkatheter (10) mit einem verlängerten flexiblen Schaft (14) mit einem proximalen und einem distalen Ende, einem Sitz (16), der mit dem proximalen Ende des Schaftes verbunden ist und eine Aufblasöffnung (20) aufweist, und einem aufblasbaren Ballon (12), der mit dem Schaft nahe dem distalen Ende des Schaftes verbunden ist; wobei der Schaft einen Aufblashohlraum (18) definiert, der eine Fluidverbindung eines Aufblasfluids zwischen der Sitzaufblasöffnung (20) und dem Ballon (12) bereitstellt; und
einen expandierbaren röhrenförmigen Gitterstent (34), der eine röhrenförmige Wandstärke definiert, befestigt um den Ballon und in einem Anfangszustand auf einen anfänglichen gefalteten äußeren Durchmesser gefaltet; wobei der Stent im wesentlichen keine Neigung aufweist, ohne eine expansive Kraft, die durch Aufblasen des Ballons (12) verursacht wird, selbst zu expandieren;
wobei der Ballon (12) zum selektiven Aufblasen von einem entleerten Zustand in einen aufgeblasenen Zustand angepasst ist, ebenso wie für späteres Entleeren; der Ballon aus einem im wesentlichen unelastischen Material ausgebildet ist; der Ballon in dem aufgeblasenen Zustand einen zylindrischen Arbeitsabschnitt (36) mit einer aufgeblasenen Querschnittsfläche aufweist, die zwischen einem Paar zylindrischer Schenkelabschnitte (48, 50) angeordnet ist, wobei die Schenkel mit dem Schaft (14) befestigt sind; der Ballon anfangs in dem entleerten Zustand ist mit einer Vielzahl von Falten in Längsrichtung; wodurch er dabei hilft, den Stent (34) durch Reibung in einer anfänglichen Position in Längsrichtung zu halten, während das Kathetersystem entlang einem vaskularen Weg vorgeschoben oder zurückgezogen wird;
wobei der Ballon (12) in seinem entleerten Zustand einen zentralen zylindrischen Bettabschnitt (42) unterhalb des gefalteten Stents (34) und eine erste und eine zweite Ballonschulter (44) definiert, die unmittelbar proximal und distal des Stents und des Ballonbettabschnittes (42) angeordnet sind; eine jede Ballonschulter (44) in einem anfänglichen entleerten Zustand eine ringförmige Form aufweist mit einer gekrümmten Wand mit ringförmigem Querschnitt und einem anfänglichen Außendurchmesser, der größer ist als der anfängliche Außendurchmesser des Stents, wodurch er dabei hilft, den Stent (34) in einer anfänglichen Position in Längsrichtung durch Reibung zurückzuhalten, während das Kathetersystem entlang einem vaskularen Weg vorgeschoben oder zurückgezogen wird, und während der Ballon (12) aufgeblasen wird; so dass der Ballon eine ausgebuchtete Bettform (42) für den Stent in dem entleerten Zustand definiert; wobei die Schultern (44) in dem entleerten Zustand angepasst sind, um das proximale und distale Ende des Stents zu schützen und die Möglichkeit eines unerwünschten Kontaktes zwischen dem proximalen und distalen Ende des Stents und einer Seitenwand des vaskularen Weges zu verringern; und
wobei der Ballon (12) angepasst ist, um sich aufzublasen und den Stent (34) auf einen größeren entfalteten Stentdurchmesser zu expandieren, wobei der Ballon den zylindrischen Arbeitsabschnitt (36) und den aufgeblasenen Zustand oberhalb eines vorab ausgewählten Übergangsdruckes ausbildet;
**dadurch gekennzeichnet, dass** der Ballon (12) in dem entleerten Zustand ein Paar zylindrische Übergangsabschnitte (46) unmittelbar proximal und distal den Schultern (44) definiert, und ein Kegelpaar (38) definiert, die sich von den Übergangsabschnitten (46) zu den Ballonschenkeln (48, 50) erstrecken; und der Ballon (12) in dem aufgeblasenen Zustand ein Paar konische Abschnitte (38) enthält, zwischen denen der zylindrische Arbeitsabschnitt (38) angeordnet ist.

2. Ballonkatheterstententfaltungssystem nach Anspruch 1, wobei der Stent (34) aus einem integralen einstückigen Metallzylinder mit einer Vielzahl von Öffnungen ausgebildet ist, um zu erlauben, dass sich der Stent von dem Anfangsdurchmesser auf den entfalteten Durchmesser expandiert.

3. Ballonkatheterstententfaltungssystem nach Anspruch 1, wobei der Stent (34) aus einem oder mehreren Drähten ausgebildet ist, die zu einem integralen nicht-einstückigen Metallzylinder angeordnet sind, der ein oder mehrere Befestigungen zwischen ausgewählten Abschnitten der Drähte aufweist.

4. Ballonkatheterstententfaltungssystem nach Anspruch 1, 2 oder 3, weiter umfassend einen Führungsdrahthohlraum (26), der angepasst ist, um verschiebbar einen verlängerten flexiblen Führungsdraht (28) aufzunehmen; wobei sich der Führungsdrahthohlraum kontinuierlich von einer distalen Führungsdrahtöffnung, die durch den Katheter distal des Ballons definiert ist, zu einer proximalen Führungsdrahtöffnung (30) nahe dem proximalen Ende des Schaftes (14) in einer über-den-Draht-Konfiguration erstreckt.

5. Ballonkatheterstententfaltungssystem nach Anspruch 1, 2 oder 3 weiter umfassend einen Führungsdrahthohlraum (26) der angepasst ist, um einen verlängerten flexiblen Führungsdraht (28) verschiebbar aufzunehmen; wobei sich der Führungsdrahthohlraum kontinuierlich von einer distalen Führungsdrahtöffnung, die durch den Katheter distal des Ballons definiert ist, zu einer proximalen Führungsdrahtöffnung (30) an einem Punkt näher zu dem distalen Ende des Katheters als dem proximalen Ende des Katheters erstreckt, in einer Schnellwechselkonfiguration.

6. Ballonkatheterstentabgabesystem nach einem der vorangehenden Ansprüche, wobei die Ballonschultern (44) und Übergangsabschnitte (46) dabei helfen, als Dilatoren zu wirken und eine glatte Bewegung nach vorne entlang einem vaskularen Weg unterstützen.

7. Ballonkatheterstententfaltungssystem nach einem der vorangehenden Ansprüche, wobei die Ballonschultern (44) dabei helfen, den Ballon (12) und den Stent (34) zu zentrieren, wenn sie durch einen zuvor implantierten Stent hindurch gehen, und ein derartiges Hindurchgehen zu erleichtern.

8. Ballonkatheterstententfaltungssystem nach einem der vorangehenden Ansprüche, wobei die Ballonschultern (44) dabei helfen, den Ballon (12) und den Stent (34) zu zentrieren, wenn der Stent zurück in einen Führungskatheter gezogen wird.

9. Ballonkatheterstententfaltungssystem nach einem der vorangehenden Ansprüche, wobei die Schultern (44) so funktionieren, dass sie eine taktile Bestätigung der Position und Größe einer Stenose bereitstellen.

## Revendications

1. Système de déploiement d'endoprothèse vasculaire de cathéter à ballonnet comprenant :
un cathéter à ballonnet (10) avec un axe flexible allongé (14) ayant des extrémités proximale et distale, un moyeu (16) apposé à l'extrémité proximale de l'axe et ayant un orifice de gonflement (20), et un ballonnet qui peut être gonflé (12) apposé à l'axe près de l'extrémité distale de l'axe ; l'axe définissant une lumière de gonflement (18) procurant une communication de fluide d'un fluide de gonflement entre l'orifice de gonflement de moyeu (20) et le ballonnet (12) ; et
une endoprothèse vasculaire d'engrènement tubulaire expansible (34) définissant une épaisseur de paroi tubulaire montée autour du ballonnet et qui est ondulée à un état initial selon un diamètre externe ondulé initial, l'endoprothèse vasculaire n'ayant sensiblement aucune tendance à s'étendre de lui-même en l'absence d'une force expansive provoquée par le gonflement du ballonnet (12) ;
dans lequel le ballonnet (12) est adapté pour un gonflement sélectif depuis un état dégonflé jusqu'à un état gonflé aussi bien que pour un dégonflement ultérieur ; le ballonnet étant formé d'un matériau sensiblement inélastique ; le ballonnet à l'état gonflé ayant une partie de travail cylindrique (36) avec une partie en coupe transversale gonflée située entre une paire de parties de branche cylindriques (48,50), les branches étant apposées à l'axe (14), le ballonnet étant initialement à l'état dégonflé ayant de multiples plis longitudinaux ; ayant tendance, de ce fait, à retenir par frottement l'endoprothèse vasculaire (34) dans une position longitudinale initiale tandis que le système de cathéter est avancé ou retiré le long d'un trajet vasculaire ;
dans lequel le ballonnet (12) à son état dégonflé définit une partie de base, cylindrique, centrale (42) en dessous de l'endoprothèse vasculaire ondulée (34) et définit un premier et un second épaulement de ballonnet (44) situés immédiatement aux extrémités proximale et distale de l'endoprothèse vasculaire et de la partie de base de ballonnet (42) ; chaque épaulement de ballonnet (44) à un état dégonflé initial ayant une forme annulaire avec une coupe transversale annulaire de paroi arquée et un diamètre externe initial plus grand que le diamètre externe initial du support, ayant tendance, de ce fait, à retenir par frottement l'endoprothèse vasculaire (34) dans une position longitudinale initiale tandis que le système de cathéter est avancé ou retiré le long d'un trajet vasculaire et tandis que le ballonnet (12) est gonflé ; de telle sorte que le ballonnet définit une forme de base de dentelée (42) pour le support à l'état dégonflé ; dans lequel lesdits épaulements (44) à l'état dégonflé sont adaptés pour protéger les extrémités proximale et distale du support et pour réduire la possibilité d'un contact indésirable entre les extrémités proximale et distale de l'endoprothèse vasculaire et une paroi latérale du trajet vasculaire ; et
dans lequel le ballonnet (12) est adapté pour gonfler et dilater l'endoprothèse vasculaire (34) jusqu'à un diamètre de support déployé plus important de sorte que le ballonnet forme ladite partie de travail cylindrique (36) et ledit état gonflé au-dessus d'une pression de transition présélectionnée ;
caractérisé à ce que le ballonnet (12) à l'état dégonflé définit une paire de parties de transition cylindriques (46) émiettement aux extrémités proximale et distale des épaulements (44) et définit une paire de conicités (38) s'étendant depuis les parties de transition (46) jusqu'aux branches de ballonnet (48,50) ; et le ballonnet (12) à l'état gonflé inclut une paire de parties coniques (38) entre lesquelles la partie de travail cylindrique (36) est située.

2. Système de déploiement d'endoprothèse vasculaire de cathéter à ballonnet selon la revendication 1, dans lequel le dit support (34) est formé d'un cylindre en métal, unitaire, intégral ayant une pluralité d'ouvertures pour permettre au support de se dilater depuis le diamètre initial jusqu'au diamètre déployé.

3. Système de déploiement d'endoprothèse vasculaire de cathéter à ballonnet selon la revendication 1, dans lequel l'endoprothèse vasculaire (34) est formée d'un ou davantage de fils disposés en un cylindre métallique non unitaire intégral ayant une ou davantage de fixations entre les parties sélectionnées des fils.

4. Système de déploiement d'endoprothèse vasculaire de cathéter à ballonnet selon la revendication 1, 2 ou 3, comprenant, en outre, une lumière de guide-fil (26) adaptée pour recevoir de manière coulissante un guide-fil flexible allongé (28) ; la lumière de guide-fil s'étendant de manière continue depuis un orifice de guide-fil distal défini par l'extrémité distale côté cathéter du ballonnet jusqu'à un orifice de guide-fil proximal (30) près de l'extrémité proximale de l'axe (14) dans une configuration lorsqu'on a le temps.

5. Système de déploiement d'endoprothèse vasculaire de cathéter à ballonnet selon la revendication 1, 2 ou 3, comprenant, en outre, une lumière de guide-fil (26) adaptée pour recevoir de manière coulissante un guide-fil flexible allongé (28) ; la lumière de guide-fil s'étendant de manière continue depuis un orifice de guide-fil distal défini par l'extrémité distale côté cathéter du ballonnet jusqu'à un orifice de guide-fil proximal (30) à un point plus proche de l'extrémité distale de cathéter que l'extrémité proximale côté cathéter, dans une configuration d'échange rapide.

6. Système de délivrance d'endoprothèse vasculaire de cathéter à ballonnet selon n'importe quelle revendication précédente, dans lequel les épaulements de ballonnet (44) et les parties de transition (46) ont tendance à agir comme des dilatateurs et à encourager un avancement sans anicroches le long d'un trajet vasculaire.

7. Système de déploiement d'endoprothèse vasculaire de cathéter à ballonnet selon n'importe quelle revendication précédente, dans lequel les épaulements de ballonnet (44) ont tendance à centrer le ballonnet (12) et l'endoprothèse vasculaire (34) lors du croisement d'un support implanté précédemment et à faciliter un tel croisement.

8. Système de déploiement d'endoprothèse vasculaire de cathéter à ballonnet selon n'importe quelle revendication précédente, dans lequel les épaulements de ballonnet (44) ont tendance à centrer le ballonnet (12) et l'endoprothèse vasculaire (34) lors du retrait de l'endoprothèse vasculaire dans un cathéter de guidage.

9. Système de déploiement d'endoprothèse vasculaire de cathéter à ballonnet selon n'importe quelle revendication précédente, dans lequel les épaulements (44) sont utiles pour fournir une confirmation tactile de la position et de la taille d'une sténose.
